Europäisches Patentamt

European Patent Office

Office européen des brevets

(11) Veröffentlichungsnummer: **0 071 658**
**B1**

# EUROPÄISCHE PATENTSCHRIFT

(45) Veröffentlichungstag der Patentschrift: **18.02.87**

(21) Anmeldenummer: **81106144.9**

(22) Anmeldetag: **05.08.81**

(51) Int. Cl.⁴: **A 61 B 17/58,** A 61 N 1/36,
A 61 F 2/32

(54) Vorrichtung zur Vitalerhaltung von Knochengewebe.

(43) Veröffentlichungstag der Anmeldung:
**16.02.83 Patentblatt 83/07**

(45) Bekanntmachung des Hinweises auf die
Patenterteilung:
**18.02.87 Patentblatt 87/08**

(84) Benannte Vertragsstaaten:
**AT BE CH DE FR IT LI NL SE**

(56) Entgegenhaltungen:
**DE-A-2 347 213
DE-A-3 003 758
US-A-4 216 548**

(73) Patentinhaber: **Kraus, Werner
Augustenstrasse 41
D-8000 München 2 (DE)**

(72) Erfinder: **Ascherl, Rudolf, Dr.med.
Höllental-Strasse 40
D-8100 Garmisch-Partenkirchen (DE)**
Erfinder: **Kraus, Werner, Dipl.-Ing.
Augustenstrasse 41/Rückgeb.
D-8000 München 2 (DE)**
Erfinder: **Lechner, Fritz, Prof. Dr.
Klarweinstrasse 29
D-8100 Garmisch-Partenkirchen (DE)**

(74) Vertreter: **von Bezold, Dieter, Dr. et al
Postfach 86 02 60
D-8000 München 86 (DE)**

Courier Press, Leamington Spa, England.

**Beschreibung**

Die vorliegende Erfindung betrifft eine Vorrichtung zur Vitalerhaltung von Knochengewebe, das an eine Oberfläche des Knochens angrenzt, an der eine Endoprothese angebracht ist, mit einer Spule, in der eine niederfrequente elektrische Wechselspannung induzierbar ist, und mit zwei drahtförmigen Elektroden, die elektrisch mit der Spule in Verbindung stehen.

Aus DE—C—1918 299 ist es bekannt, daß niederfrequente Wechselströme Knochengewebe zum Wachstrum anregen und vital erhalten können. Dies ist besonders für Endoprothesen, wie Hüftgelenkkopfprothesen oder Gelenk-Totalprothesen von Bedeutung, da bei solchen Prothesen erfahrungsgemäß nach einiger Zeit eine Lockerung eintritt und eine Nachoperation erforderlich wird, wenn nicht das Schwinden und die Devitalisierung des Knochens, die für diese Lockerung verantwortlich sind, verhindert werden.

Es ist aus der DE—A—26 11 744 bekannt, daß die Devitalisierung und das Schwinden des Knochens dadurch verhindert werden können, daß man die in kraftschlüssigem Kontakt mit dem Knochen, stehende Endoprothese mit einer Elektrodenanordnung versieht, die mit einer Spule verbunden ist, in der durch niederfrequentes magnetisches Wechselfeld eine entsprechende Wechselspannung induzierbar ist. Die Elektrodenanordnung enthält Elektroden, die abwechselnd mit dem einen und anderen Anschluß der Spule verbunden sind, so daß eine flächige Stimulation der Knochenoberfläche bewirkt wird, die den Knochen fest um die Prothese wachsen läßt und vital erhält, so daß keine Lockerung der Prothese eintritt.

Die aus der DE—A—26 11 744 bekannten Endoprothesen setzen voraus, daß sie ohne eine nennenswerte Zwischenlage von Knochenzement oder dergleichen implantiert werden, da die auf der Prothesenoberfläche angeordneten Elektroden sonst das Knochengewebe nicht stimulieren können. Die meisten Chirurgen arbeiten heutzutage jedoch mit Knochenzement, da dies eine sofortige, feste Verbindung zwischen dem Knochen und der implantierten Prothese gewährleistet, so daß die Prothese sehr bald nach der Operation belastet werden kann. Die Verwendung von Knochenzement hat aber nicht nur den Nachteil, daß simulierende Prothesen der aus der DE—A—26 11 744 bekannten Art nicht verwendet werden können, sondern auch den Nachteil, daß der einen polymerisierbaren Kunststoff enthaltende Knochenzement bei der Aushärtung sehr heiß wird und das anliegende Knochengewebe dadurch schädigen kann, was wiederum die Lockerung der Prothese begünstigt.

Der vorliegenden Erfindung liegt die Aufgabe zugrunde, eine Vorrichtung zur Vitalerhaltung von Knochengewebe anzugeben, die auch bei Verwendung gewöhnlicher Endoprothesen, d.h. Endoprothesen ohne Stimulationselektroden, und auch bei der Implantation von (gewöhnlichen) Endoprothesen mit Knochenzement verwendet werden kann.

Diese Aufgabe wird durch eine Vorrichtung mit den Merkmalen des Oberbegriffes des Anspruchs 1 gelöst, die erfindungsgemäß die im kennzeichnenden Teil des Anspruchs 1 aufgeführten Merkmale aufweist.

Weiterbildungen und vorteilhafte Ausgestaltungen der Vorrichtung gemäß der Erfindung sind Gegenstand von Unteransprüchen.

Die Vorrichtung gemäß der Erfindung läßt sich bei der Implantation gewöhnlicher (d.h. elektrodenloser) Endoprothesen mit oder ohne Knochenzement verwenden und gewährleistet, daß der Knochenbereich, der in kraftübertragender Verbindung mit der Endoprothese steht, auch über lange Zeiten vital erhalten wird. Ferner heilen eventuelle Schäden, die durch die Erhitzung des Knochenzements im Knochen entstanden sind, schnell aus.

Wenn, was vorteilhafterweise der Fall ist, das die Elektroden während der Implantation halternde und positionierende Flächengebilde aus einem resorbierbaren Werkstoff, wie chirurgischem Nahtmaterial, besteht, ergibt sich der weitere Vorteil, daß der durch die Elektroden zum Wachstum angeregte Knochen in die durch die Resorption entstandenen Hohlräume hineinwächst, so daß eine sehr feste Verbindung zwischen dem Knochen und dem Implantat gewährleistet ist.

Im folgenden werden Ausführungsbeispiele der Erfindung unter Bezugnahme auf die Zeichnung näher erläutert.

Es zeigen:

Fig. 1 eine perspektivische Ansicht einer ersten Ausführungsform der Vorrichtung gemäß der Erfindung;

Fig. 2 eine schematische Querschnittsansicht eines Teiles der Vorrichtung gemäß Fig. 1;

Fig. 3 einen Teil eines menschlichen Oberschenkelknochens, in dem eine Hüftgelenkskopfprothese und eine Vorrichung der in Fig. 1 und 2 dargestellten Art implantiert sind;

Fig. 4 eine perspektivische Darstellung einer zweiten Ausführungsform der Erfindung und

Fig. 5 eine Schaltbild des elektrischen Teiles der Vorrichtung gemäß Fig. 4.

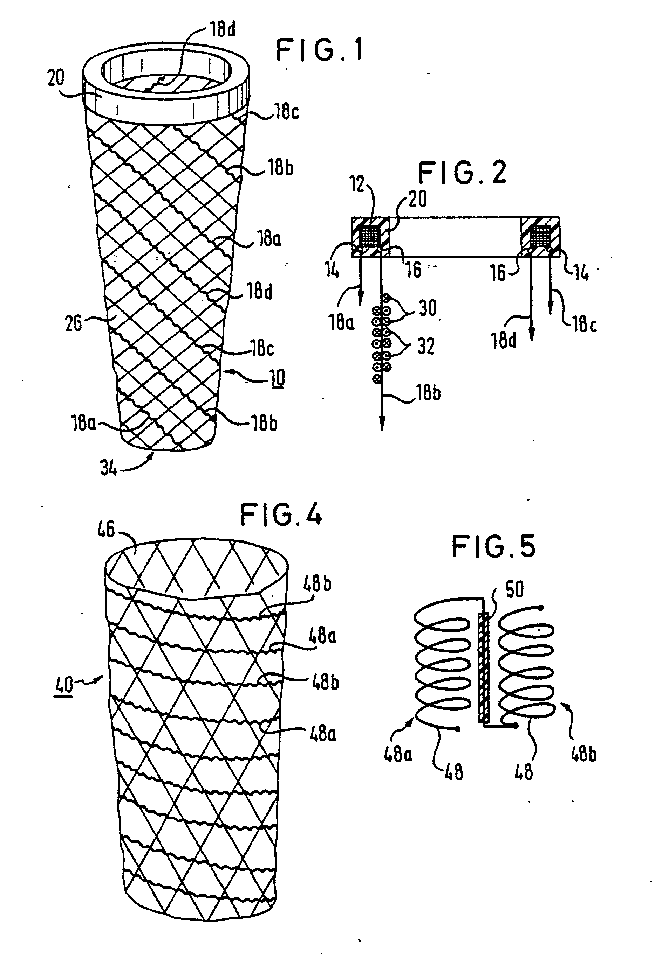

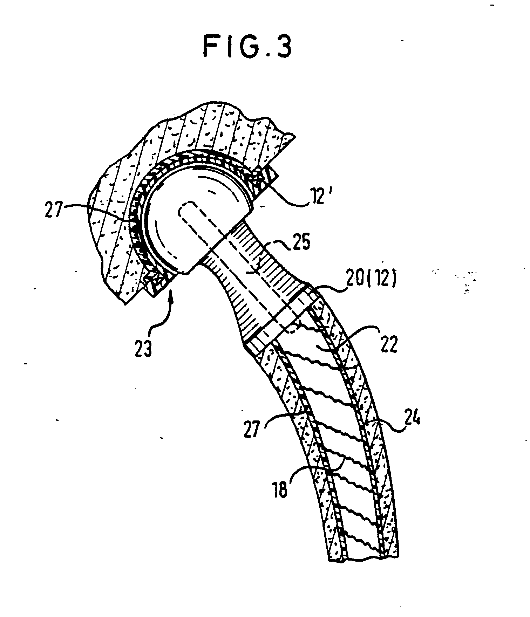

Die in Fig. 1 sind 2 dargestellte Vorrichtung 10 enthält eine Solenoidspule 12 mit zwei Anschlüssen 14, 16, an die jeweils zwei drahtförmige Elektroden 18a, 18b, 18c und 18d angeschlossen sind. Die Spule 12 ist mit einem Mantel 20 aus einem gewebeverträglichen Kunststoff, wie PTFE umgeben, der vorzugsweise eine ausreichende Elastizität hat, um sich dem Umriß eines Prothesenschaftes 22 (Fig. 3) anpassen zu können und andererseits druckfest genug ist, um zwischen den Koff einer Prothese und das angrenzende Stirnende eines Knochens 24, in den die Hüftkopfprothese implantiert ist, eingefügt werden zu können.

Die Anschlüsse 14 und 16 der Spule sind in den Mantel 20 eingebettet, wie aus Fig. 2 ersichtlich ist, und die Elektroden 18a bis 18c treten an vier Stellen des Mantels aus, die in Umfangsrichtung der Spule Abstände von etwa 90 Grad voneinander haben.

Die Spule 12 kann zum Beispiel 200 Windungen eines mit lack isolieraten Kupfer- oder Silberdrahtes enthalten.

Die Elektroden 18 bestehen aus einem gewebeverträglichen Metall, wie Osteosynthesemetall, insbesondere einer Kobalt-Chrom-Molybdänlegierung, oder aus einer Platin-Iridium-Legierung und hat vorzugsweise die Form von flexiblen Litzen.

Die Elektroden 18 sind in einem Flächengebilde 26 gehalten, das vorzugsweise aus einem biologisch resorbierbarem Material, wie chirurgischem Nahtmaterial besteht. Das Flächengebilde 26 kann vorzugsweise ein rohrförmiges Gewebe mit schräg zur Rohrachse verlaufenden Fäden sein, wie es in Fig. 1 durch die sich kreuzenden geraden Linien dargestellt ist (die Elektroden sind zur besseren Unterscheidung durch dickere etwas wellig gezeichnete Linien dargestellt). Ein solches Gewebe mit schrägem Fadenverlauf läßt sich in Axialrichtung strecken und in Umfangsrichtung der Form des Prothesenschaftes 22 anpassen.

Die Elektroden 18 sind vorzugsweise umsponnen, und zwar, wie es in Fig. 2 stark schematisch dargestellt ist, mit einer durch Kreuzchen bezeichneten ersten Windung aus einem biologisch verträglichen und stabilen Faden 30 aus zum Beispiel PTFE und einem zwischen die Windungen dieses Fadens gewickelten Faden 32 aus einem biologisch resorbierbaren Material, wie chirurgischen Nahtmaterial.

Das schlauchartige Flächengebilde 26 kann an dem der Spule 20 abgewandten Ende 34 geschlossen oder offen sein.

Bei einer Operation wird die Vorrichtung 10 nach dem Öffnen und Ausbohren des Implantationsbereiches, beispielsweise des Oberschenkelknochens 24, zum Einsetzen auf eine konusförmige Vorrichtung gesteckt, deren Form dem Bohrwerkzeug entspricht. Es kann auch das Werkzeug selbst verwendet werden, wenn durch eine Zwischenlage, zum Beispiel aus einer dünnen PTFE-Folie gewährleistet ist, daß die Vorrichtung 10 nicht beschädigt wird. Die Vorrichung 10 wird nun in den Implantationsbereich, z.B. in die Markhöhle des Oberschenkelknochens eingeschoben und durch Drehen der Einführungsvorrichtung (bei Verwendung des Bohrwerkzeuges durch Drehen in Rückwärtsrichtung) zur satten Anlage an die mit der Prothese zusammenwirkende Oberfläche des Knochens gebracht. Dabei verankert sich das Flächengebilde 26 mit den Elektroden 18 an den Knochenbälkchen der Markhöhle oder dergleichen.

Nun wird die Prothese entweder unmittelbar oder nach Einfüllen von Knochenzement in das Innere der Vorrichtung 10 eingesetzt. Die Elektroden 18 kommen auf diese Weise unmittelbar zwischen Knochen und den Prothesenkörper bzw. den Knochenzement zu liegen. Zwei bis drei Wochen nach der Operation ist das resorbierbare Material des Flächengebildes 26 und der Elektrodenumspinnung resorbiert und die zurückbleibenden Metallelektroden 18 sind fest in mineralisiertes Bindegewebe eingewachsen. Die verbleibende Umspinnung aus dem stabilen Fadenmaterial 30 verhindert einen Kurzschluß der Elektroden durch die gewöhnliche aus Metall bestehende Prothese aus denjenigen Stellen, wo die Prothese direkt an der Vorrichtung 10 anliegt.

Wenn die Prothese aus Keramik oder mit Calciumphosphat beschichteten Metall besteht oder die Isolation auf andere Weise gewährleistet ist, kann die Umspinnung mit dem stabilen Faden 30 entfallen. Die Elektroden brauchen dann überhaupt nicht umsponnen zu werden. Eine resorbierbare Umspinnung ist jedoch immer vorteilhaft, da sie einen Elektronenkurzschluß verhindert, wenn sich z.B. beim Einsetzen der Vorrichtung 10 Falten im Flächengebilde 26 bilden.

Zur Konzentration des elektrischen Feldes in der Spule können in der Prothese ein oder mehrere magnetisch hochpermeable Kerne vorgesehen sein.

Bei der Ausführungsform gemäß Fig. 4 werden die Induktionsspule und die Elektroden durch ein und denselben Leiter 48 gebildet. Dieser Leiter bildet zwei ineinandergeschachtelte Wendeln 48a und 48b, die durch eine isolierte Verbindung 50 so hintereinandergeschaltet sind, daß zwischen benachbarten Windungen der beiden Wendeln 48a und 48b jeweils die volle Spannung liegt, die durch ein niederfrequentes magnetisches Wechselfeld in jeder der Drahtwendeln 48a und 48b induziert wird. Die Wendeln sind wieder in einem flexiblen, leicht verformbaren schlauchartigen Gebilde 46 verankert und die Vorrichtung 40 gemäß Fig. 4 wird ähnlich verwendet wie die Vorrichtung gemäß Fig. 1.

Die erfindungsgemäße Vorrichtung kann auch in Verbindung mit einem Hüftgelenkpfannenimplantat 23 (Fig. 3) Verwendung finden. Das Schlauchartige oder sackartige Flächengebilde erhält dann eine näherungsweise kugelkalottenförmige Gestalt und die Elektroden können dann z.B. spiralartig verlaufen.

Die Elektroden können auch im wesentlichen parallel zur Schlauchachse verlaufen, sie haben dann vorzugsweise jedoch eine zickzackförmige Gestalt, damit das schlauchartige Gebilde gestreckt werden kann, ohne die Elektroden zu stark zu beanspruchen.

Anstelle eines gewebten Flächengebildes kann auch ein nichtgewebtes Flächengebilde, z.B. in Form eines Faserfilzes oder einer Folie, die vorteilhafterweise perforiert ist, verwendet werden.

Das Flächengebilde, das die Elektroden haltert und postitioniert, kann auch gewirkt sein. Es kann auch die Form eines dünne, flexiblen, Blattes haben, also z.B. aus einem rechteckigen Stück eines Gewebes aus chirurgischen Nahtmaterial bestehen.

Wenn das Flächengebilde blattförmig ist, hat die Iduktionsspule vorteilhafterweise die Form eines dünnen Stabes oder Plättchens, so daß sie im Knochenzement zwischen dem Knochen und der Prothese untergebracht werden kann. Die Elektroden können strahlenförmig von der in der Mitte des Blattes angeordneten Induktionsspule ausgehen, so daß das Blatt nach Belieben zugeschnitten werden kann.

Das Flächengebilde 26 bzw. 46 ist zweckmäßigerweise so porös oder weitmaschig, daß die fixierende Wirkung des Knochenzements nicht beeinträchtigt wird sondern der Knochenzement durch das Flächengebilde zur Knochenoberfläche gelangen kann.

Bei einer Hüftgelenkprothese kann im Kopf und Schaft der Schenkelhalsprothese ein Magnetkern aus hochpermeablem Material angeordnet sein, wie in Fig. 3 bei 25 dargestellt ist. Ein solcher Magnetkern gewährleistet eine gute Einkopplung äußerer magnetischer Wechselfelder sowohl in die Spule 12 der der Schenkelhalsprothese zugeordneten Vorrichtung 10 als auch in die Spule 12', die der der Gelenkpfannenprothese 23 zugeordneten Vorrichtung. Der Magnetkern kann auch aus einem permanentmagnetischen Material bestehen und teilweise oder ganz magnetisiert sein, sein Feld Induziert dann bei Bewegung des Hüftgelenks eine Wechselspannung in der Spule 12' und ggf. auch in der Spule 10.

## Patentansprüche

1. Implantierbare Vorrichtung zur Vitalerhaltung von Knochengewebe, welches an eine Oberfläche eines Knochens angrenzt, an der eine Endoprothese angebracht ist, mit einer Spule (12), in der eine niederfrequente elektrische Wechselspannung induzierbar ist, und mit mindestens zwei drahtförmigen Elektroden (18a, 18b, 18c, 18d), die elektrisch mit der Spule in Verbindung stehen, dadurch gekennzeichnet, daß die drahtförmigen Elektroden (18a, 18b, 18c, 18d) durch ein dünnes, flexibles, zwischen die Oberfläche des Knochens (24) und die Endoprothese einlegbares Flächengebilde (26) aus elektrisch isolierendem Werkstoff im Abstand voneinander gehalten sind.

2. Implantierbare Vorrichtung zur Vitalerhaltung von Knochengewebe, das an eine Oberfläche eines Knochens angrenzt, an der eine Endoprothese angebracht ist, mit einem wendelförmigen Leiter (48), welcher eine Spule, in der eine niederfrequente elektrische Wechselspannung induzierbar ist, und zwei drahtförmige Elektrodenteile (48a, 48b), zwischen denen die induzierte Spannung auftritt, bildet, dadurch gekennzeichnet, daß die drahtförmigen Elektrodenteile (48a, 48b) durch ein dünnes, flexibles, zwischen die Oberfläche des Knochens und die Endoprothese einlegbares schlauchförmiges Flächengebilde (46) aus elektrisch isolierendem Werkstoff im Abstand voneinander gehalten sind.

3. Vorrichtung nach Anspruch 1 oder 2, dadurch gekennzeichnet, daß das Flächengebilde (26, 46) aus einem biologisch resorbierbaren Material besteht.

4. Vorrichtung nach Anspruch 1, 2 oder 3, dadurch gekennzeichnet, daß das Flächengebilde (26, 46) aus Fasermaterial besteht.

5. Vorrichtung nach Anspruch 1, 2, 3 oder 4, dadurch gekennzeichnet, daß das Flächengebilde blattförmig ist.

6. Vorrichtung nach Anspruch 1, 2, 3 oder 4, dadurch gekennzeichnet, daß das Flächengebilde (46) die Form eines Schlauches hat.

7. Vorrichtung nach Anspruch 6, dadurch gekennzeichnet, daß die Spule (12) am einen Ende des schlauchförmigen Flächengebildes (46) angeordnet ist.

8. Vorrichtung nach Anspruch 2, dadurch gekennzeichnet, daß der wendelförmige Leiter (48) zwei mit gleichem Windungssinn in Reihe geschaltete Drahtwendeln (48a, 48b) enthält, welche nach Art einer zweigängigen Schraube ineinandergeschachtelt sind und die Elektrodenteile bilden.

9. Vorrichtung nach Anspruch 6 oder 7, dadurch gekennzeichnet, daß die Elektroden (18a bis 18d) die Form von mehrgängigen Wendeln oder Spiralen haben. 10. Vorrichtung nach einem der vorhergehenden Ansprüche, dadurch gekennzeichnet, daß die Elektroden (18, 48) mit einem resorbierbaren Faden (32) umsponnen sind.

11. Vorrichtung nach einem der Ansprüche 1 bis 10, dadurch gekennzeichnet, daß die Elektroden mit einem nichtresorbierbaren Faden (30) aus einem biologisch verträglichen Material umsponnen sind.

12. Vorrichtung nach Anspruch 10 und 11, dadurch gekennzeichnet, daß der resorbierbare Faden (32) zwischen die Windungen des nichtresorbierbaren Fadens (30) gewickelt sind.

## Revendications

1. Dispositif implantable pour maintenir vital du tissu osseux contigu à la surface d'un os auquel est appliquée une endoprothèse, comportant une bobine (12) dans laquelle peut être induite une tension électrique alternative de basse fréquence, ainsi qu'au moins deux électrodes filiformes (18a, 18b, 18c, 18d) qui sont reliées électriquement à la bobine, caractérisé en ce que les électrodes filiformes (18a, 18b, 18c, 18d) sont maintenues à distance l'une de l'autre par un corps (26) plat (de surface étendue pour une faible épaisseur) mince et flexible, qui peut être disposé entre la surface de l'os (24) et l'endoprothèse et est formé d'un matériau électriquement isolant.

2. Dispositif implantable pour maintenir vital du tissu osseux contigu à une surface d'un os auquel est appliquée une endoprothèse, comportant un conducteur hélicoïdal (48) formant une bobine, dans laquelle peut être induite une tension électrique alternative de basse fréquence, et deux parties d'électrodes (48a, 48b) filiformes, entre lesquelles s'établit la tension induite, caractérisé en ce que les parties d'électrodes (48a, 48b) filiformes sont maintenues à distance l'une de l'autre par un corps (46) plat (de surface étendue pour une faible épaisseur) qui est mince, flexible et constitué de matériau électriquement isolant, ce corps étant conformé en tube souple et étant susceptible d'étre disposé entre la surface de l'os et l'endoprothèse.

3. Dispositif selon la revendication 1 ou 2, caractérisé en ce que le corps plat (26, 46) est fait d'un matériau biologiquement résorbable.

4. Dispositif selon la revendication 1, 2 ou 3, caractérisé en ce que le corps plat (26, 46) est en matériau fibreux.

5. Dispositif selon la revendication 1, 2, 3 ou 4, caractérisé en ce que le corps plat est en forme de feuille.

6. Dispositif selon la revendication 1, 2, 3 ou 4, caractérisé en ce que le corps plat (46) affecte la forme d'un tube souple.

7. Dispositif selon la revendication 6, caractérisé en ce que la bobine (12) est disposée à une extrémité du corps plat (46) conformé en tube souple.

8. Dispositif selon la revendication 2, caractérisé en ce que le conducteur hélicoïdal (48) comprend deux hélices de fil (48a, 48b) ayant le même sens d'enroulement, qui sont imbriquées à la façon des filets d'une vis à filet double, les hélices étant connectées en série et formant les parties d'électrodes.

9. Dispositif selon la revendication 6 ou 7, caractérisé en ce que les électrodes (18a à 18d) affectent la forme d'éléments hélicoïdaux ou spiralé multiples.

10. Dispositif selon une des revendications précédentes, caractérisé en ce que les électrodes (18, 48) sont guipés ou recouvertes d'une autre manière d'un fil (32) résorbable.

11. Dispositif selon une des revendications 1 à 10, caractérisé en ce que les électrodes sont guipées ou recouvertes d'une autre manière d'un fil (30) non résorbable en matériau biocompatible.

12. Dispositif selon les revendications 10 et 11, caractérisé en ce que le fil résorbable (32) est enroulé entre les spires du fil non résorbable (30).

**Claims**

1. Implantable device for maintaining the vitality of osseous tissue adjoining a surface of a bone to which an endoprothesis is attached, comprising a coil (12) in which a low-frequency electrical AC voltage can be induced and at least two wire-shaped electrodes (18a, 18b, 18c, 18d) which are in electrical connection with the coil, characterized in that the wire-shaped electrodes (18a, 18b, 18c, 18d) are mounted spaced from each other by a thin flexible sheet structure (26) of electrically insulating material insertable between the surface of the bone (24) and the endoprothesis.

2. Implantable device for maintaining the vitality of osseous tissue adjoining a surface of a bone to which an endoprothesis is attached, comprising a spiral conductor (48) which forms a coil in which a low-frequency electrical AC voltage can be induced and two wire-shaped electrode portions (48a, 48b) between which the induced voltage occurs, characterized in that the wire-shaped electrode portions (48a, 48b) are mounted spaced apart by a thin flexible tube-like sheet structure (46) of electrically insulating material insertable between the surface of the bone and the endoprothesis.

3. Device according to claim 1, characterized in that the sheet material (26, 46) consists of a biologically resorbable material.

4. Device according to claim 1, 2 or 3, characterized in that the sheet structure (26, 46) consists of fibrous material.

5. Device according to claims 1, 2, 3 or 4, characterized in that the sheet structure is sheet-shaped.

6. Device according to claim 1, 2, 3 or 4, characterized in that the sheet structure (46) has the form of a flexible tube.

7. Device according to claim 6, characterized in that the coil (12) is disposed at the one end of the tubular sheet structure (46).

8. Device according to claim 2, characterized in that the spiral conductor (48) includes two wire spirals (48a, 48b) which are connected in series with the same convolution direction and which are nested in each other in the manner of a double-threaded screw and form the electrode portions.

9. Device according to claim 6 or 7, characterized in that the electrodes (18a to 18d) have the form of multi-threaded helices or spirals.

10. Device according to any one of the preceding claims, characterized in that the electrodes (18, 48) are surrounded by a resorbable thread (52).

11. Device according to any one of claims 1 to 10, characterized in that the electrodes are surrounded by a non-resorbable thread (30) of a biologically neutral material.

12. Device according to claims 10 and 11, characterized in that the resorbable thread (32) is wound between the turns of the non-resorbable thread (30).

FIG.1

FIG.2

FIG.4

FIG.5

# FIG.3